# EUROPEAN PATENT APPLICATION

(11) **EP 3 272 393 A1**
(43) Date of publication of application: **24.01.2018**
(21) Application number: 17187432.4
(22) Date of filing: 20.10.2010
(51) Int. Cl.: A61N 1/36, A61N 1/04

(54) **PROGRAMMABLE ELECTRICAL STIMULATION OF THE FOOT MUSCLES**

(30) Priority: 15.01.2010 US 687935
(62) Divisional of application: 10843391.3
(71) Applicant: StimMed LLC, Buffalo, NY 14221 (US)
(72) Inventor: CZYRNY, James Joseph, Amherst, NY 14226 (US); KAPLAN, Robert E., Buffalo, NY 14222-1147 (US)
(74) Representative: Heinze, Ekkehard

(57) **Abstract**

A device for delivering electrical stimulation to muscles of a foot (31) of a patient, the device comprising one or more power sources; a signal generator (410) for generating electrical current, the signal generator remotely programmable by a physician monitoring the patient, wherein the electrical current is for causing the muscles to contract or for disturbing pain signals communicated by the muscles to brain; at least two electrodes (430, 435) in communication with the signal generator (410) for delivery of the electrical current to the foot, wherein the at least two electrodes are adapted to be located at two or more of medial ankle at location of posterior tibial nerve, lateral ankle at location of sural nerve, and anterior ankle at a location of anterior tibial nerve; and an accelerometer in communication with the signal generator, wherein the signal generator ceases to generate the electrical current if the accelerometer communicates movement of the patient and restarts to generate electrical current after a pre-programmed period of inactivity by the patient is communicated by the accelerometer.

## Description

### BACKGROUND OF THE INVENTION

### 1-FIELD OF THE INVENTION

The present invention pertains generally to the field of the electrical stimulation of muscles for prevention of thrombosis and for pain management and, more particularly, to electrical stimulation of muscles of the foot.

### 2- DESCRIPTION OF RELATED ART

Electrical stimulation of muscles and nerves by applying electrodes over the skin is currently used for enhancing blood circulation and reducing blood clots and for scrambling the pain signal that reach the brain in order to manage pain.

Patients undergoing surgery, anesthesia and extended periods of bed rest or other inactivity are often susceptible to a condition known as deep vein thrombosis, or DVT. DVT is a clotting of venous blood in the lower extremities or pelvis. This clotting occurs due to the absence of muscular activity required to pump the venous blood in the lower extremities, local vascular injury or a hypercoaguble state. The condition can be life-threatening if a blood clot migrates to the lung, resulting in pulmonary embolism (PE), or otherwise interferes with cardiovascular circulation. More generally, venous thromboembolic disease (VTED) is a cause of significant morbidity and mortality for individuals immobilized after orthopedic surgery, due to neurologic disorders, even during prolonged travel, and a variety of other conditions.

Since 1954 it has been known that prolonged dependency stasis, a state imposed by airplane flights, automobiles trips and even attendance at the theater may bring on thrombosis. In 1977, it was shown that trips as short as three to four hours could induce DVT and PE.

DVT and related conditions may be controlled or alleviated by assisting blood circulation (venous return) in the muscles.

Current approaches to prophylaxis include mechanical compression using pneumatic compression devices, anticoagulation therapy and electrical stimulation of the muscles. Pneumatic compression equipment is often too cumbersome for mobile patients, or during prolonged travel. Anticoagulation therapy carries the risk of bleeding complications and must be started several days in advance to be effective. Electric stimulation has advantages over the other two methods in that it can be started at the time prophylaxis is needed and can be portable using DC current sources.

A number of U.S. patents teach various methods of applying electrical stimulation to the calf muscle for the prevention of DVT. These include Powell, III, U.S. Patent No. 5,358,513, Tumey, U.S. Patent No. 5,674,262, Dennis, III, U.S. Patent No. 5,782,893, Katz, U.S. Patent No. 5,643,331, and Katz, U.S. Patent No. 6,002,965.

U.S. Patent No. 6,615,080 to Unsworth et al. provides a method for preventing DVT, PE, ankle edema and venostasis and a device that includes a single channel sequential neuromuscular electrical stimulation (NMES) unit. The NMES unit is battery powered and can be programmed to deliver a particular stimulus profile. In order to simplify the patient's ability to properly apply the NMES device, the stimulator generates biphasic symmetrical square wave pulses with stimulus parameters demonstrated to result in optimum venous blood flow. The stimulus profile included a stimulus frequency fixed at 50 pulses per second, a stimulus duration of 300 microseconds, a ramp up time of 2 seconds, a ramp down time of 2 seconds, and a stimulus cycle set at 12 seconds on and 48 seconds off. Once set in advance by the doctor, manufacturer or user, the patient adjusts the intensity, using a stimulus intensity dial, to the point needed to produce a minimally visible or palpable muscle contraction. The output leads of the stimulator are attached through a conductor to electrodes of various types including, self-adherent surface electrodes. These electrodes are of opposite polarity and create an electrical potential difference between themselves and the tissue that separates them. The frequency and electrical characteristics of electrical impulses applied to the patient is referred to as the electrical stimulation routine.

In published but abandoned U.S. Patent Application Publication No. 2006/0085047A, a variation of Unsworth et al. provided a method of automatically controlling the delivery of, single channel NMES of the plantar muscle, in response to the sensing of motion of the foot or leg. In the published application, the stimulation is turned off during walking or running to prevent slips or falls and to reduce power consumption of the unit that provides the stimulation.

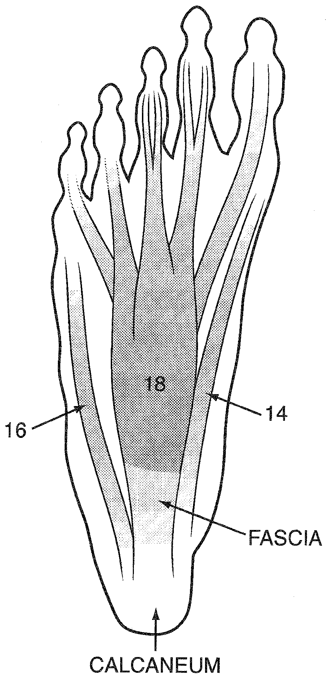

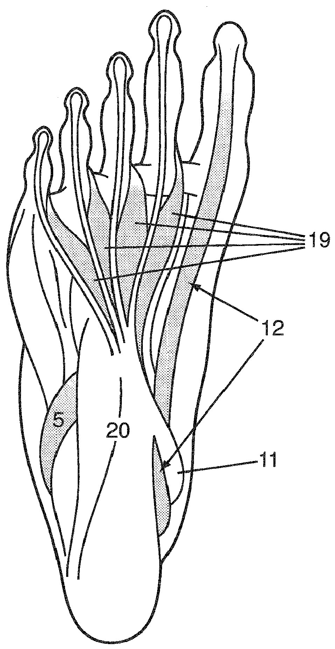

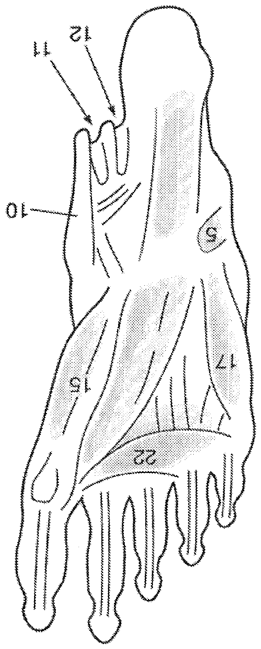

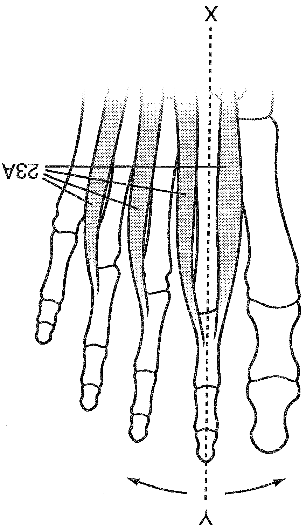

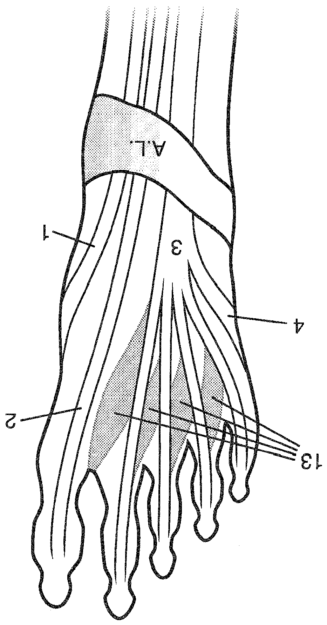

Figure 1A, Figure 1B, Figure 1C, Figure 1D and Figure 1E show muscles of the sole of a foot.

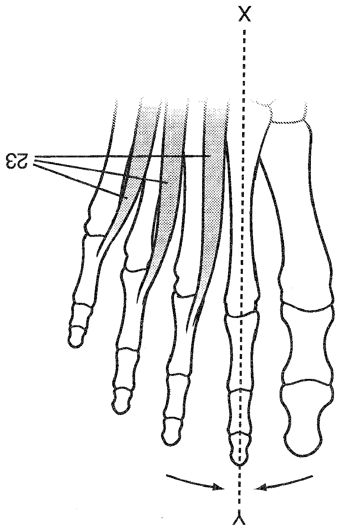

There are four layers of muscles in the sole of the foot. After the skin of the plantar region and the fatty tissue have been removed, an expansion of fibrous tissue known as the plantar fascia is visible. If this is also taken away, the first layer of muscles is exposed, consisting of abductor pollicis (14), flexor brevis digitorum (18), and the abductor minimi digiti (16) (Figure 1A). The second layer, situated under the first, consists of the tendons of the flexors longus digitorum,(11) proprius, and pollicis. (12). On the outer side of the foot, the tendon of the peroneus longus (5) passes beneath the flexor accessories (20). To complete the layer the muscles flexor accessorius (20) and the lumbricales (19) must be named (Figure 1 B). The third plantar layer consists of the tendon of tibialis posticus (10), the flexor brevis pollicis (15), the adductor pollicis (21), the flexor brevis minimi digiti (17), and, running across the foot, the transversus pedis (22). The sheath of the peroneus longus (5), and the plantar ligament, are also found in this layer (Figure 1C). The fourth layer (Figure 1F), consists of three interossei (23), one on the inner side of the second toe, and the others one each on the inner side of the third and fourth toes.

They draw to the central line XY, called the "central muscular action line," or the "line of muscular action." The first layer (Figure 1E) on the dorsal surface consists of the tendons of the tibialis anticus (1), extensor proprius pollicis (2), extensor longus digitorum (3), and the tertius peroneus (4). The muscles of the extensor brevis digitorum (13), after passing under the extensor longus digitorum (3), divide into four tendons, and aid in the extension of the toes. The second layer (Figure 1D) consists of four interossei (23a), fixed on the outer side of the second, third, and fourth toes, and draw from the" central muscular action line " XY, and one on inner side of second toe drawing to line XY.

Muscles of the foot are also divided into two groups of plantar (internal, external, central), which pertains to the sole of the foot, and dorsal which indicates the back muscles behind the plantar muscles.

The dorsal group includes:
13. Extensor brevis digitorum. First layer.
23a. Interossei dorsal (4). Second layer.
The plantar group includes:
14. Abductor pollicis. Internal first layer.
15. Flexor brevis pollicis. Internal third layer.
16. Abductor minimi digiti. External first layer.
17. Flexor brevis minimi digiti. External third layer.
18. Flexor brevis digitorum. Central first layer.
19. Lumbricales. Central second layer.
20. Flexor accessorius. Central second layer.
21. Adductor pollicis. Central third layer.
22. Transversus pedis. Central third layer.
23. Interossei plantar (3). Fourth layer.

The location and function of each muscle is further described below.
13. The extensor brevis digitorum arises in the upper outer side of the heel-bone, and, broadening out, it passes under the extensor longus digitorum, when it divides into four tendons that go forward and are inserted in the bases of the first phalanges. Its action is to aid the extension of the toes and to counteract the tendency of obliquity of the extensor longus digitorum.
14. The abductor pollicis rises on the inner posterior region of the os calcis, and is inserted in the first phalanx of the great toe. Its action is to abduct the big toe away from the central line of the foot to the imaginary line that forms the centre of the body. By this action the great toes would be brought closer together.
15. The flexor brevis pollicis comes from the second row of the tarsus, and is inserted to the base of the first phalanx.
16. The abductor minimi digiti arises from the outside of the os calcis, and goes forwards to the external side of the first phalanx of the little toe. Its action is to draw the little toe away from the middle line of the foot.
17. The flexor brevis minimi digiti has origin in the sheath of the Peroneus longus and the base of the fifth metatarsal bone, and is inserted in the first phalanx of the little toe. Its action is to flex the little toe.
18. The flexor brevis digitorum, from the heel-bone and the plantar fascia, draws down the toes, and is inserted in the second phalanges of the four toes.
19. The four lumbricales are affixed to the inner side of the four toes. Their action is to draw the toes in to the inner side of the foot.
20. The flexor accessorius extends from the os calcis to the second, third, and fourth toes. In contraction it counteracts the obliquity of the flexor longus digitorum, hence its name.
21. The adductor pollicis arises from the sheath of the peroneus longus and the third and fourth metatarsals, and is inserted in the first phalanx of the great toe on the outer side. Its action is to adduct, or draw, the great toe to the central line of the foot.
22. The transversus pedis goes across the foot, and is inserted in the phalanx of the great toe. Its office is to adduct, or draw, the big toe to the line of the foot termed the "line of muscular action."
23. The three plantar interossei are situated between the bones of the toes on the inner side, and draw to the central line the three outer toes.
23a. The four interossei, on the dorsal surface of the foot, are situated on the outer side of the bones of the toes, and draw the third and fourth toes away from the central line of muscular action. The two interossei on either side of the second toe draw away from the axis of the toe either to the outer or inner side of the foot respectively.

The foot is provided with two kinds of nerves - - those that supply the skin with sensory branches, and the other sort that give motor impressions to the muscles. The posterior tibial and the anterior tibial nerves come from the sciatic nerve, the former giving branches to the muscles in passing down to the inner side of the ankle. The posterior tibial then divides into external plantar nerves and internal plantar nerves, that supply the toes and sole of the foot. The anterior tibial nerves supply the dorsum of the foot as well as the outer side of the leg.

Under the skin are found pads of fat, at the heel and toes especially.

The muscles of the foot are further classified as either intrinsic or extrinsic. The intrinsic muscles are located within the foot and cause movement of the toes. These muscles are flexors (plantar flexors), extensors (dorsiflexors), abductors, and adductors of the toes. Several intrinsic muscles also help support the arches of the foot. The extrinsic muscles are located outside the foot, in the lower leg. The powerful calf muscle is among them. Most of these muscles have long tendons that cross the ankle, to attach on the bones of the foot and assist in movement.

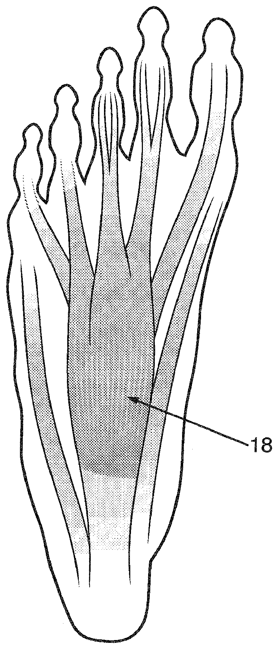

Figure 2 shows the flexor digitorum brevis muscle.

This muscle is responsible for flexing the four smaller toes. It lies in the middle of the sole of the foot, immediately above the central part of the plantar aponeurosis, with which it is firmly united. Its deep surface is separated from the lateral plantar vessels and nerves by a thin layer of fascia. It arises by a narrow tendon, from the medial process of the tuberosity of the calcaneus, from the central part of the plantar aponeurosis, and from the intermuscular septa between it and the adjacent muscles. It passes forward, and divides into four tendons, one for each of the four lesser toes.

Of the other muscle of the first layer, the abductor digiti minimi (abductor minimi digiti, abductor digiti quinti) is a muscle which lies along the lateral border of the foot, and is in relation by its medial margin with the lateral plantar vessels and nerves. Its function is to flex and abduct the fifth (little) toe. The last muscle of the first layer, abductor pollicis is like the abductor digiti minimi except that it lies along the lateral inside border of the foot and connects to the big toe.

Figure 3A and Figure 3B show placement of electrodes as disclosed by Unsworth et al., U.S. Patent No. 6,615,080.

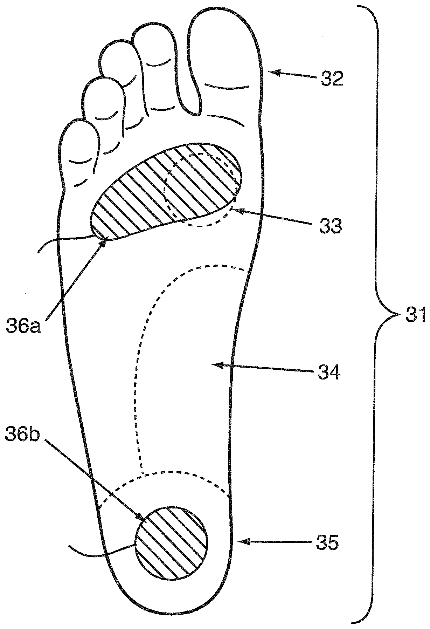

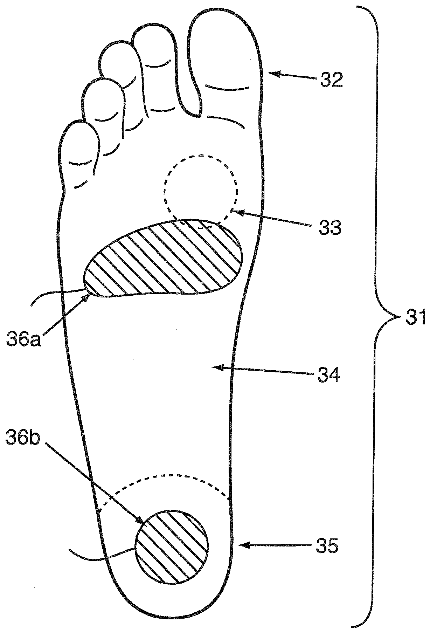

Figure 3A illustrates a sole of a foot 31. Toes 32, ball 33, arch 34, and heel 35 are shown in the drawing. Electrodes 36a, 36b are located in an area over intrinsic muscles on the plantar surface of the foot, or proximal to them, for example on or around the ball of the foot 33, and over or proximal to the heel 35. In Figure 3A electrodes 36a and 36b are placed that deliver the electrical impulses generated by the NMES device 30. Figure 3B shows an alternate area 36a' at which an electrical impulse can be delivered. In some embodiments of the Unsworth invention, the electrode 36a occupies only the area of the ball of the foot, while other embodiments include elliptical electrodes having their major axis normal to the longitudinal axis of the foot 31.

As shown in Figure 3A and Figure 3B, the Unsworth issued patent applies one electrode over or proximal to the heel and the other over the intrinsic muscles on the plantar surface of the foot, for example, on or around the ball of the foot. In Unsworth the intensity of the electrical stimulation required is only that necessary to create a slight visible muscle twitch of the foot muscles, or a minimally visible or palpable muscle contraction. By stimulating in this manner, blood pooling in the calf veins was prevented.

Electrical stimulation is also utilized for pain management. The most common form of electrical stimulation used for pain management is transcutaneous electrical nerve stimulation (TENS) therapy, which provides short-term pain relief. Electrical nerve stimulation and electrothermal therapy are used to relieve pain associated with various conditions, including back pain. For example, intradiscal electrothermal therapy (IDET) is a treatment option for people with low back pain resulting from intervertebral disc problems. In TENS therapy for pain management, a small, battery-operated device delivers low-voltage electrical current through the skin via electrodes placed near the source of pain. The electricity from the electrodes stimulates nerves in the affected area and sends signals to the brain that "scramble" normal pain perception. TENS is not painful and has proven to be an effective therapy to mask pain.

### SUMMARY OF THE INVENTION

Aspects of the present invention provide systems, devices and methods for providing neuromuscular electrical stimulation (NMES) to muscles of the foot. One aspect provides a single channel stimulator device that includes an electrical signal generator for producing a wave pattern of variable frequency, duration, intensity, ramp time and on-off cycle. The stimulator device further includes surface electrodes for being positioned over the foot muscles and attached to the signal generator. The signal generator is programmed to stimulate the foot muscles. The programming is adjusted to reduce pooling of the blood in the soleal veins of the calf and enhance venous blood flow to prevent DVT, to enhance venous blood flow for the post-thrombotic syndrome patient, to expedite wound healing, to reduce neuropathic pain of the foot and ankle, chronic musculoskeletal pain of the ankle and foot, and acute post-operative foot and ankle pain, and to prevent muscular atrophy of the foot muscles.

In some aspects of the present invention, the electrodes are arranged on the heel and the mid-section or arch of the foot. This arrangement is appropriate for systems, devices and methods of the present invention that contribute to (1) enhanced venous blood flow to prevent DVT, (2) enhanced venous blood flow for the post-thrombotic syndrome patient and (3) prevention of muscular atrophy of the foot muscles.

As Figure 3A and Figure 3B of the drawings show, in Unsworth, one electrode is located on the heel while the second electrode targets the ball of the foot.

Aspects of the present invention place the second electrode in the arch of the foot. This location targets the flexor digitorum brevis muscle. This muscle is the largest muscle; it is close to the skin and is separated from the lateral plantar vessels and nerves by a thin layer of fascia, and it is responsible for flexing the four smaller toes. Because it is a larger muscle, it generates more circulation when it is stimulated and because it is closer to the skin it is more accessible by the electrode. Moreover, one end of this muscle is located at the heel and the electrical pulse may be conducted through the length of the muscle and the nerves that control it.

The ball of the foot and its vicinity are separated from the skin with a thicker layer of fat and the skin is generally more calloused in that area. The arch of a normal foot is seldom calloused and has a relatively thin skin. Moreover, the lumbricals, which are located under the ball lie in the second layer of foot muscles which is located deeper and further from the skin. Lumbricals are much smaller than the flexor digitorum brevis and control the same 4 small toes. Except, the motion generated by the lumbricals is an adduction motion, which is not as extensive as a flexing motion, and generally would not generate as much circulation.

The electrodes are located on the heel and the bottom of the mid-foot region or the arch. The active electrode is located at the mid-foot region and the ground electrode is located at the heel.

Aspects of the present invention further provide systems, devices and methods that contribute to (1) enhanced wound healing, (2) reduction of the neuropathic pain of the foot and ankle, (4) reduction of the chronic musculoskeletal pain of the ankle and foot, and (5) reduction of the acute post-operative foot and ankle pain. These aspects of the present invention provide pain relief by generating a tapping feeling that results from intermittent electrical stimulation of the muscle. For reduction of neuropathic pain, chronic musculoskeletal pain, acute post-op pain, and wound healing, the electrodes are placed at the level of the main ankle bones called the medial malleolus and the lateral malleolus. For both electrodes, the connection site would be just below the malleolus. For other indications, the electrodes are located on the sole of the foot.

Aspects of the present invention provide a device for delivering electrical stimulation to muscles of a foot of a patient. The device includes one or more power sources, a signal generator for generating electrical current, and electrodes in communication with the signal generator for delivery of the electrical current to the foot. The electrical current is for causing the muscles to contract, and the electrodes are adapted to be located on a heel of the foot and on an arch of the foot.

Aspects of the present invention provide a device for delivering electrical stimulation to muscles of a foot of a patient. The device includes one or more power sources, a signal generator for generating electrical current, and electrodes in communication with the signal generator for delivery of the electrical current to the foot. The electrical current is for disturbing pain signals communicated by the muscles to brain, and the electrodes are connected anteriorly to ankle to stimulate peroneal nerve of the foot. The electrodes may be adapted to be located at two or more of medial ankle at location of posterior tibial nerve, lateral ankle at location of sural nerve, and anterior ankle at e location of anterior tibial nerve.

Aspects of the present invention provide a method for enhancing venous blood flow to prevent deep vein thrombosis, enhancing venous blood flow for post-thrombotic syndrome patients, and preventing muscular atrophy of foot muscles. The method includes connecting electrodes to a foot of the patient, and applying electrical current of a programmable waveform, intensity, frequency and duration to the foot muscles through the electrodes. A ground electrode is connected to a heel of the foot, and a positive electrode is connected to an arch of the foot.

Aspects of the present invention provide a method for enhancing wound healing, reducing neuropathic pain of the foot and ankle, reducing chronic musculoskeletal pain of the ankle and foot, and reducing acute post-operative foot and ankle pain. The method includes connecting electrodes to a foot of the patient, and applying electrical current of a programmable waveform, intensity, frequency and duration to the foot muscles through the electrodes. The electrodes are connected anteriorly to the ankle to stimulate peroneal nerve of the foot. The electrodes may be connected at two or more of just below the medial malleolus at posterior tibial nerve, at lateral malleolus at sural nerve, and at anterior ankle at anterior tibial nerve.

### BRIEF DESCRIPTION OF THE DRAWINGS

Figure 1A, Figure 1B, Figure 1C, Figure 1D, Figure 1E and Figure 1F show muscles of the sole of a foot.
Figure 2 shows the flexor digitorum brevis muscle.
Figure 3A and Figure 3B show placement of electrodes as disclosed by Unsworth et al., U.S. Patent No. 6,615,080.
Figure 4 shows a device for providing electrical stimulation to the foot, according to aspects of the present invention.
Figure 5 shows placement of electrodes on the foot, according to aspects of the present invention.
Figures 6A, 6B and 6C show placement of electrodes on the foot for pain management, according to further aspects of the present invention.
Figure 7 shows a flowchart of a method of increasing circulation, according to aspects of the present invention.
Figure 8 shows a flowchart of a method of pain management, according to aspects of the present invention.
Figure 9 shows a device for providing electrical stimulation to the foot, according to aspects of the present invention.

### DETAILED DESCRIPTION OF THE INVENTION

Aspects of the present invention provide an improved system, device and method of administering electrical stimulation to the muscles of the foot.

Aspects of the present invention provide a programmable electrical pulse generator for delivering an electrical current of mild and tolerable intensity to the muscles of the foot that results in a mild contraction of the muscles. In various aspects of the present invention, the contraction may be accomplished by placing surface electrodes on the soles of the feet or at the ankles. When placed on the soles, the active surface electrodes are placed over the larger muscles of the first layer that are closer to the surface of the skin and in an area where callousing of the skin and the fat layer are minimal such as the mid-foot and arch area. The ground electrodes may be placed over or proximal to the heel. By stimulating the foot muscles in this manner, blood pooling in the calf veins is prevented. When placed on the side or top of the ankles, the surface electrodes stimulate the posterior tibial, anterior tibial, or the sural nerves. By stimulating the peripheral nerves with the arrangement of electrodes around the ankles, pain management and improved wound healing may be achieved.

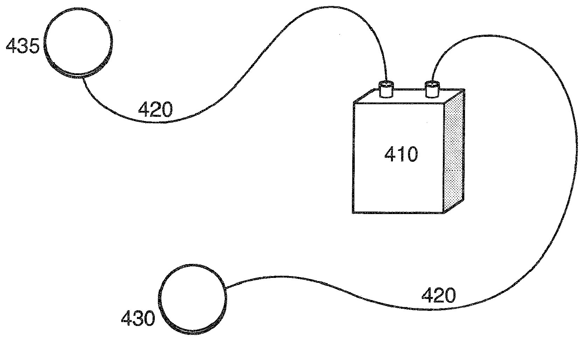

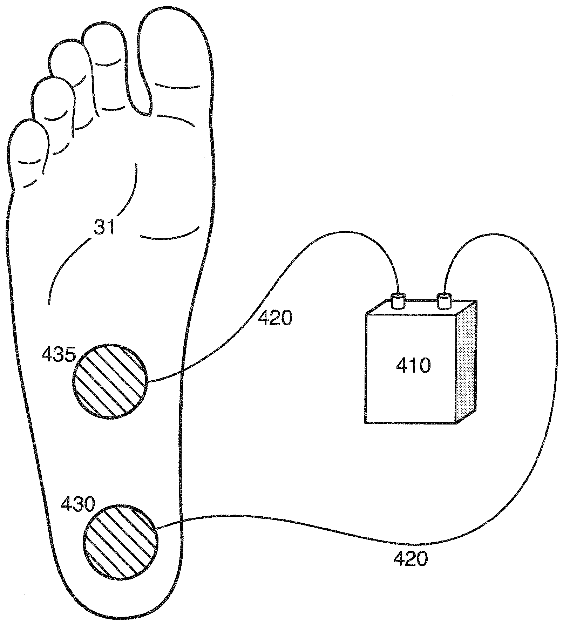

Figure 4 and Figure 9 show a device for providing electrical stimulation to the foot, according to aspects of the present invention.

The device 400 includes a generator 410, connecting wires 410 and electrodes 430 and 435. The electrodes are connected to the generator via the connecting wires. The generator 410 is a programmable electrical stimulation signal generation device. The electrodes 430 and 435 may be interchangeable and their polarity is determined according to their connection to the generator 410. The electrodes are self adhesive or otherwise attachable to skin.

Various aspects of the present invention may be implemented in footwear and accessories to footwear such as shoes, socks and stockings. They may be carried in a pocket or pouch in a item of footwear, with conductors connecting the a stimulus generating portion of the device to electrodes placed on the skin. The electrodes may vary in shape and size and may be self-adhering of the type utilized for TENS devices. Moreover, if at least one of the electrodes includes a power source, then the electrodes may be wirelessly in communication with the signal generator. In that situation, the signal generator may be located closer to the hands and head of the user allowing him to more easily adjust the intensity and other parameters of the stimulation. In the case of wireless control, the electrodes must be connected together, outside the body, to create a closed circuit with the passage through the muscles. Further, the signal generator may be remotely programmable by a physician monitoring the patient.

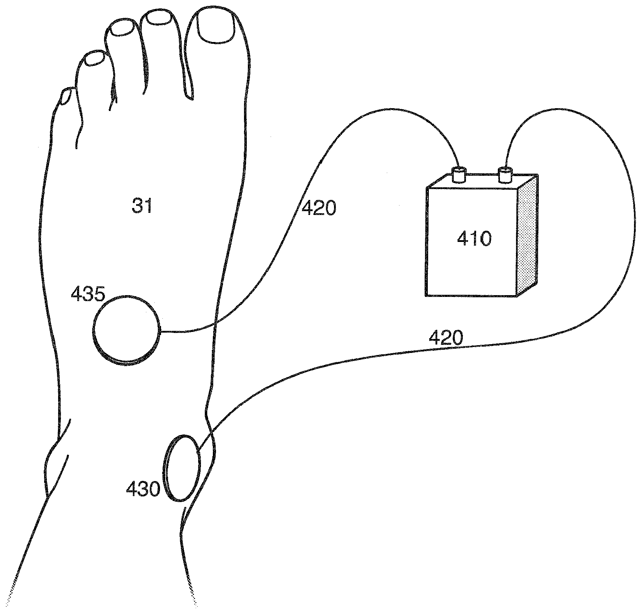

Figure 5 shows placement of electrodes on the foot, according to aspects of the present invention.

The electrodes 430 and 435 are located on the foot 31 such that one electrode attaches to the heel and the other is attached to the mid-section or the big arch of the foot. In the arch area the skin is not calloused and the fat layer under the skin is minimal. In one aspect, the heel electrode 430 is the ground electrode and the arch electrode is the active or positive electrode.

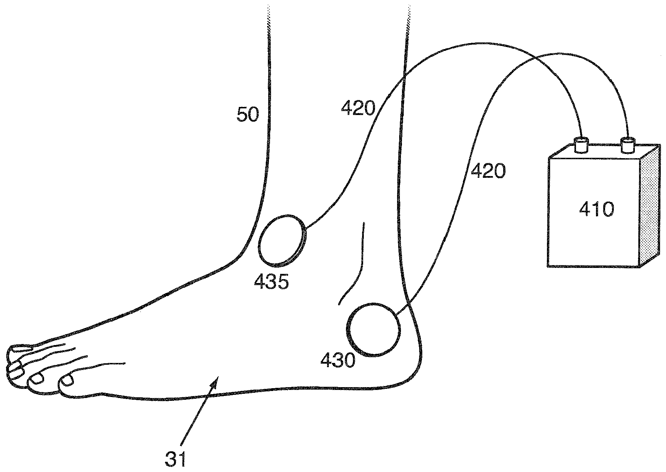

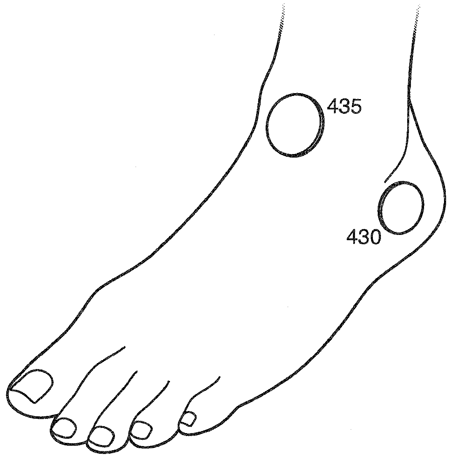

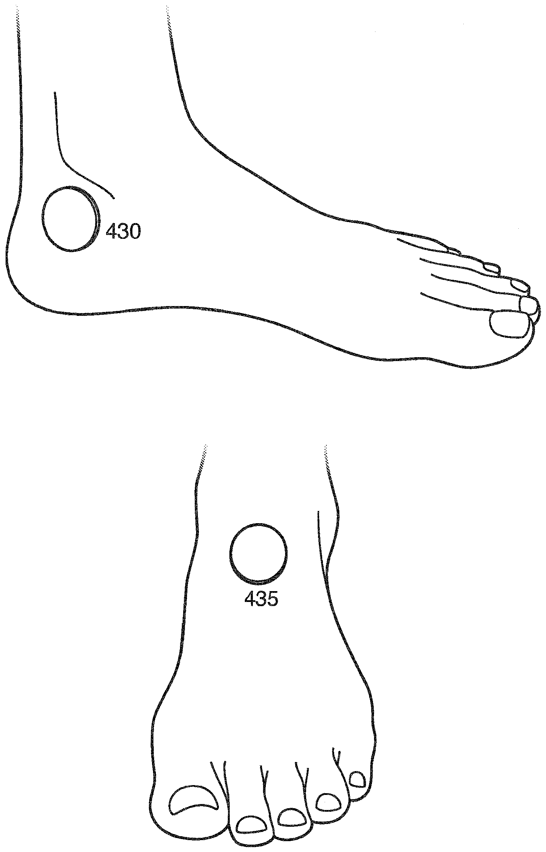

Figures 6A, 6B and 6C show placement of electrodes on the ankles, according to aspects of the present invention.

The placement of the ankle electrodes is chosen to optimally stimulate the posterior tibial, anterior tibial, and sural nerves of the leg 50. This in turn will provide the maximum therapeutic effect for pain management, enhancing wound healing, and preventing muscle atrophy. These electrodes may be located at the area of the peroneal motor nerve which is also referred to as the anterior tibial nerve. In one aspect, the electrodes would be placed just lateral to the tendon of tibialis anterior and just proximal to the malleoli. Figure 6B provides the ankle showing anterior electrode placement (435) and lateral electrode placement (430). Figure 6C provides a line drawing of the ankle showing anterior electrode placement (435) and medial electrode placement (430).

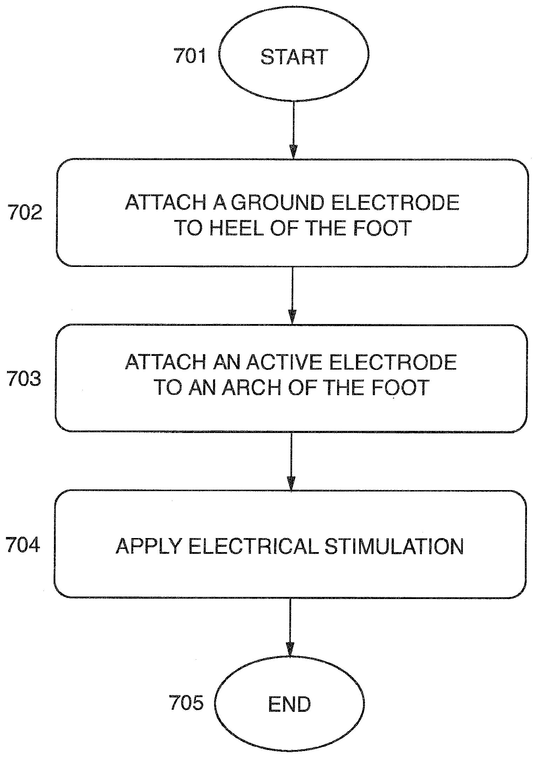

Figure 7 shows a flowchart of a method of increasing circulation, according to aspects of the present invention.

The method begins at 701. At 702, one electrode, for example the ground electrode, is connected to heel of a foot. At 703, the other electrode, for example the active electrode, is connected to a mid-section or arch of the foot. At 704, electrical stimulation is applied to the muscles of the foot through the attached electrodes. At 705, the method ends.

In variations of this method, the electrical stimulation may be periodically or continuously adjusted according to readout of parameters from the patient or according to decision of a physician or the patient himself.

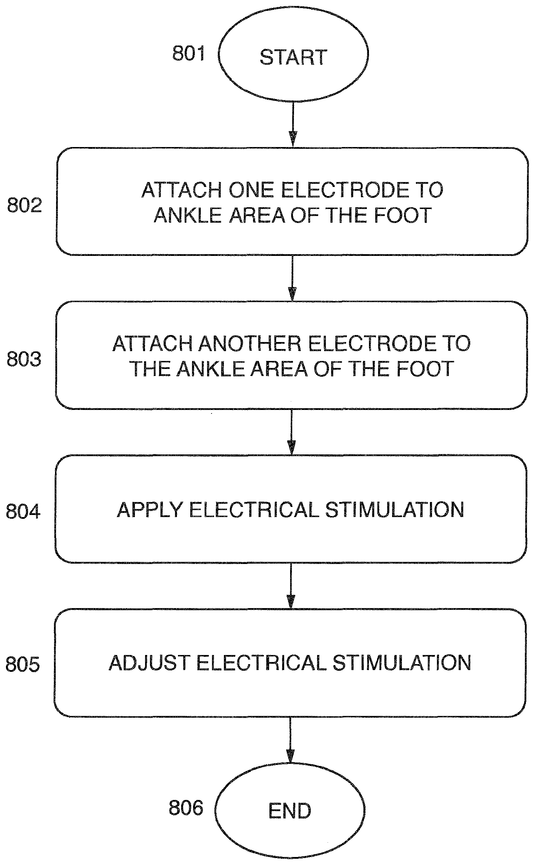

Figure 8 shows a flowchart of a method of pain management, according to aspects of the present invention.

The method begins at 801. At 802, one electrode, for example the ground electrode, is connected above the ankle of a foot. At 803, the other electrode, for example the active electrode, is connected to below the ankle of the foot. At 804, electrical stimulation is applied to the muscles of the foot through the attached electrodes. At 805, the electrical stimulation is adjusted. At 806, the method ends.

The present invention has been described in relation to particular examples, which are intended to be illustrative rather than restrictive, with the scope and spirit of the invention being indicated by the following claims and their equivalents.

### Aspects of the invention

1. A device for delivering electrical stimulation to muscles of a foot of a patient, the device comprising:
   one or more power sources;
   a signal generator for generating electrical current; and
   electrodes in communication with the signal generator for delivery of the electrical current to the foot,
   wherein the electrical current is for causing the muscles to contract, and
   wherein the electrodes are adapted to be located on a heel of the foot and on an arch of the foot.
2. The device of aspect 1, further comprising:
   an input and output interface for receiving input parameters for programming the device and echoing current parameters to a user;
   a processor for controlling the electrical current generated by the signal generator according to the input parameters; and
   a storage medium in communication with the processor and the input and output interface for storing the input parameters.
3. The device of aspect 2, wherein the input parameters are received from the user, from a monitoring device for monitoring the patient, from the patient or from any combination.
4. The device of aspect 2, wherein the input parameters are adjusted to enhance venous blood flow to prevent deep vein thrombosis, enhance venous blood flow for the post-thrombotic syndrome patients, and prevent muscular atrophy of the foot muscles.
5. The device of aspect 1, wherein the one or more power sources are proximal to the signal generator, the device further comprising:
   conductors coupled between the electrodes and the signal generator for delivering the electrical current from the signal generator to the electrodes.
6. The device of aspect 1,wherein the signal generator is a single channel sequential neuromuscular electrical stimulation (NMES) device.
7. The device of aspect 1,
   wherein the electrical current delivered is in the form of a biphasic symmetrical square wave,
   wherein the square wave has a frequency of substantially equal to 50 pulses per second, and
   wherein the electrical current delivered has an intensity of up to 20 milliamperes.
8. The device of aspect 1, wherein the electrical current is in the form of a pulse with a ramp up time of substantially equal to 2 seconds and a ramp down time of substantially equal to 2 seconds.
9. The device of aspect 1, further comprising:
   an accelerometer in communication with the signal generator,
   wherein the signal generator ceases to generate the electrical current if the accelerometer communicates movement of the patient and restarts to generate electrical current after a pre-programmed period of inactivity by the patient is communicated by the accelerometer.
10. A device for delivering electrical stimulation to muscles of a foot of a patient, the device comprising:
   one or more power sources;
   a signal generator for generating electrical current; and
   electrodes in communication with the signal generator for delivery of the electrical current to the foot,
   wherein the electrical current is for disturbing pain signals communicated by the muscles to brain,
   wherein the electrodes are connected anteriorly to ankle to stimulate peroneal nerve of the foot.
11. The device of aspect 10, wherein the electrodes are adapted to be located at two or more of medial ankle at location of posterior tibial nerve, lateral ankle at location of sural nerve, and anterior ankle at e location of anterior tibial nerve.
12. The device of aspect 10, further comprising:
   an input and output interface for receiving input parameters for programming the device and echoing the input parameters to a user;
   a processor for controlling the electrical current generated by the signal generator according to the input parameters; and
   a storage medium in communication with the processor and the input and output interface for storing the input parameters.
13. The device of aspect 12, wherein the input parameters are received from the user or from a monitoring device for monitoring the patient, from the patient, or from a combination.
14. The device of aspect 13, wherein the input parameters are adjusted to enhance wound healing, reduce neuropathic pain of the foot and ankle, reduce chronic musculoskeletal pain of the ankle and foot, and reduce acute post-operative foot and ankle pain.
15. The device of aspect 10, wherein the one or more power sources are proximal to the signal generator, the device further comprising:
   conductors coupled between the electrodes and the signal generator for delivering the electrical current from the signal generator to the electrodes.
16. A method for enhancing venous blood flow to prevent deep vein thrombosis, enhancing venous blood flow for post-thrombotic syndrome patients, and preventing muscular atrophy of foot muscles, the method comprising:
   connecting electrodes to a foot of the patient, and
   applying electrical current of a programmable waveform, intensity, frequency and duration to the foot muscles through the electrodes,
   wherein a ground electrode is connected to a heel of the foot, and
   wherein a positive electrode is connected to an arch of the foot.
17. The method of aspect 16, wherein the electrical current is adjusted responsive to condition of the patient.
18. A method for enhancing wound healing, reducing neuropathic pain of the foot and ankle, reducing chronic musculoskeletal pain of the ankle and foot, and reducing acute post-operative foot and ankle pain, the method comprising:
   connecting electrodes to a foot of the patient, and
   applying electrical current of a programmable waveform, intensity, frequency and duration to the foot muscles through the electrodes,
   wherein the electrodes are connected anteriorly to the ankle to stimulate peroneal nerve of the foot.
19. The method of aspect 18, wherein the electrical current is adjusted responsive to condition of the patient.
20. The method of aspect 18, wherein the electrodes are connected at two or more of just below the medial malleolus at posterior tibial nerve, at lateral malleolus at sural nerve, and at anterior ankle at anterior tibial nerve.

## Claims

1. A device for delivering electrical stimulation to muscles of a foot (31) of a patient, the device comprising:
one or more power sources;
a signal generator (410) for generating electrical current, the signal generator remotely programmable by a physician monitoring the patient, wherein the electrical current is for causing the muscles to contract or for disturbing pain signals communicated by the muscles to brain;
at least two electrodes (430, 435) in communication with the signal generator (410) for delivery of the electrical current to the foot, wherein the at least two electrodes are adapted to be located at two or more of medial ankle at location of posterior tibial nerve, lateral ankle at location of sural nerve, and anterior ankle at a location of anterior tibial nerve; and
an accelerometer in communication with the signal generator, wherein the signal generator ceases to generate the electrical current if the accelerometer communicates movement of the patient and restarts to generate electrical current after a pre-programmed period of inactivity by the patient is communicated by the accelerometer.

2. The device of claim 1, further comprising:
an input and output interface for receiving input parameters for programming the device and echoing current parameters to a user;
a processor for controlling the electrical current generated by the signal generator (410) according to the input parameters; and
a storage medium in communication with the processor and the input and output interface for storing the input parameters.

3. The device of claim 2, wherein the input parameters are received from the user, from a monitoring device for monitoring the patient, from the patient, or from any combination thereof.

4. The device of claim 2, wherein the input parameters are adjusted to enhance venous blood flow to prevent deep vein thrombosis, enhance venous blood flow for the post-thrombotic syndrome patients, or prevent muscular atrophy of the foot muscles.

5. The device of claim 2, wherein the input parameters are adjusted to enhance wound healing, reduce neuropathic pain of the foot and ankle, reduce chronic musculoskeletal pain of the ankle and foot, or reduce acute post-operative foot and ankle pain.

6. The device of claim 1, wherein the one or more power sources are proximal to the signal generator (410), the device further comprising:
conductors coupled between the electrodes and the signal generator for delivering the electrical current from the signal generator to the electrodes.

7. The device of claim 1,
wherein the electrical current delivered is in the form of a biphasic symmetrical square wave,
wherein the square wave has a frequency of substantially equal to 50 pulses per second, and
wherein the electrical current delivered has an intensity of up to 20 milliamperes.

8. The device of claim 1, wherein the electrical current is in the form of a pulse with a ramp up time of substantially equal to 2 seconds and a ramp down time of substantially equal to 2 seconds.

9. The device of claim 1, wherein the signal generator (410) is a single channel sequential neuromuscular electrical stimulation device.
